# FASCICULE DE BREVET EUROPEEN

(11) **EP 0 490 778 B1**
(45) Date de publication et mention de la délivrance du brevet: **28.09.1994**
(21) Numéro de dépôt: 91403411.1
(22) Date de dépôt: 16.12.1991
(51) Int. Cl.: C07C 327/36, C07D 333/18, C08G 18/38, C08G 18/52, C08G 75/04, G02B 1/04

(54) **Composés soufrés et polymères obtenus à partir de ces composés soufrés**
Schwefelverbindungen und daraus hergestellte Polymere
Sulphur compounds and polymers obtained therefrom

(30) Priorité: 14.12.1990 FR 9015978
(43) Date de publication de la demande: 17.06.1992
(73) Titulaire: RHONE-POULENC CHIMIE, 92408 Courbevoie Cédex (FR)
(72) Inventeur: Lavault, Sylvie, F-69003 Lyon (FR); Velleret, Gérard, F-75011 Paris (FR)
(74) Mandataire: Ricalens, François

(56) Documents cités:
- EP-A- 0 024 919
- EP-A- 0 351 073
- DE-A- 1 668 711
- DE-B- 1 116 658
- US-A- 3 428 598
- US-A- 3 630 991
- US-A- 4 609 762

## Description

La présente invention concerne de nouveaux composés soufrés qui sont des mercaptals d'esters de thioalcanols.

Elle concerne également l'utilisation de ces esters soufrés pour préparer des polymères présentant un indice de réfraction supérieur à 1,50.

De nombreuses voies ont été proposées pour atteindre ce résultat.

L'une des plus prometteuses est l'utilisation de monomère comportant un ou plusieurs atomes de soufre.

EP-A-351 073 décrit des composés thio ou mercapto utilisés dans la préparation de polymère présentant un indice de réfraction supérieur à 1,50, lesdits polymères étant aussi employès dans la fabrication de lentilles ophtalmologiques.

DE-B-116 658 et EP-A-024 919 décrivent des composés soufrés qui sont proches des produits intermédiaires utilisables pour la préparation des composés de l'invention.

Toutefois, aucun des 3 documents cités ci-dessus ne décrit des mercaptals d'esters de thioalcanols.

La présente invention consiste tout d'abord dans les composés soufrés de formule générale (I) : 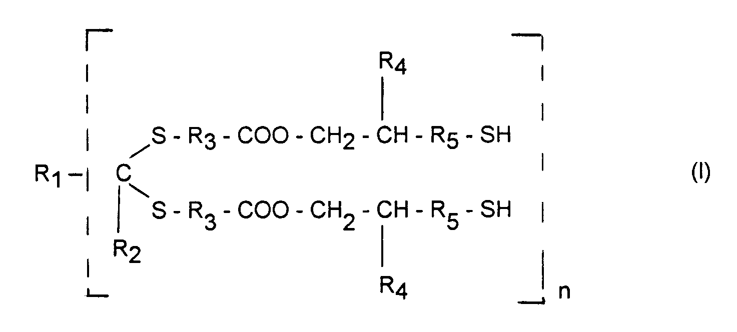
dans laquelle :
- n représente 1 ou 2
- R₁ représente :
   . lorsque n = 1 :
      + un radical alkyle, linéaire ou ramifié, ayant 1 à 6 atomes de carbone,
      + un radical phényle,
      + un radical thiényle-2,
   . lorsque n = 2 :
      + un valentiel simple,
      + un radical alkylène, linéaire ou ramifié ayant 1 à 4 atomes de carbone,
      + un radical paraphénylène, métaphénylène ou orthophénylène,
      + un radical thiophénediyle-2,4, thiophènediyle-2,5 ou thiophènediyle-2,3,
- R₂ représente :
   . un atome d'hydrogène,
   . un radical alkyle, linéaire ou ramifié ayant 1 à 6 atomes de carbone,
   . un radical phényle,
- R₃ représente un radical alkylène, linéaire ou ramifié, ayant 1 à 3 atomes de carbone,
- R₄ représente :
   . un atome d'hydrogène,
   . un radical SH,
- R₅ représente :
   . un lien valentiel simple,
   . un radical méthylène.

Parmi les composés soufrés de formule (I), on préfère ceux pour lesquels :
- n représente 1 ou 2,
- R₁ représente :
   . lorsque n = 1 :
      + un radical alkyle, linéaire ou ramifié, ayant 1 à 4 atomes de carbone,
      + un radical phényle,
      + un radical thiényle-2,
   . lorsque n = 2 :
      + un lien valentiel simple,
      + un radical alkylène, linéaire ou ramifié, ayant 1 à 4 atomes de carbone,
      + un radical paraphénylène,
      + un radical thiophènediyle-2,5,
- R₂ représente :
   . un atome d'hydrogène,
   . un radical alkyle, linéaire ou ramifié, ayant 1 à 4 atomes de carbone,
   . un radical phényle,
- R₃ représente un radical méthylène,
- R₄ représente un atome d'hydrogène,
- R₅ représente un lien valentiel simple.

A titre d'exemples non limitatifs de composés soufrés de formule (I), on peut citer :
- le tétrakis(mercapto-2 éthoxycarbonylméthylthio)-1,1,2,2 éthane,
- le bis[bis(mercapto-2 éthoxycarbonylméthylthio)méthylène]-1 ,4 benzène,
- le (2-thiényl)-4 dithia-3,5 pimélate de bis(mercapto-2 éthyle),
- le phényl-4 dithia-3,5 pimélate de bis(mercapto-2 éthyle).

Les composés soufrés de formule (I) peuvent être préparés par estérification de l'acide carboxylique correspondant de formule (II) : 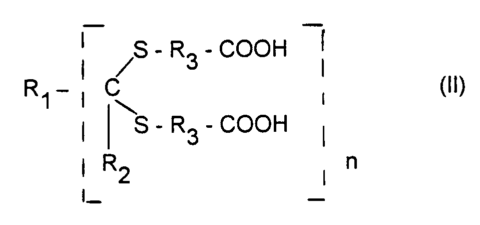
dans laquelle les symboles R₁, R₂, R₃ et n ont les significations indiquées précédemment pour la formule (I), avec un thioalcanol de formule (III) : 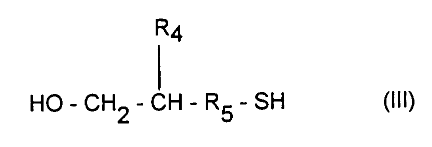
dans laquelle les symboles R₄ et R₅ ont les significations indiquées précédemment pour la formule (I).

La réaction d'estérification se fait habituellement en présence des catalyseurs connus de cette réaction, tels que les acides protoniques.

Généralement l'estérification est effectuée dans un solvant organique formant un azéotrope avec l'eau, de manière à permettre l'élimination de l'eau formée dans la réaction.

Comme exemples illustratifs de tels solvants, on peut citer le toluène, le benzène, les xylènes.

La température est le plus souvent de 50°C à 150°C et pourra être en pratique la température de reflux du solvant utilisé.

Les quantités des composés de formules (II) et (III) peuvent être très variables, mais en général on utilise au moins la quantité stoechiométrique de thioalcanol de formule (III) par rapport aux fonctions carboxyliques du composé de formule (II) et de préférence on met en oeuvre un excès de thioalcanol (III) par rapport aux fonctions carboxyliques du composé (II).

Les acides carboxyliques de formule (II) sont connus pour certains d'entre-eux. Ils peuvent être préparés par réaction d'un aldéhyde ou d'une cétone de formule générale (IV) : 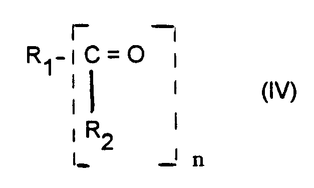
dans laquelle les symboles R₁ et R₂ ont les significations indiquées précédemment pour les composés soufrés de formule (I) et n représente 1 ou 2, avec un acide mercapto-carboxylique de formule générale (V) :

HS-R₃-COOH (V)

dans laquelle R₃ représente un radical alkylène, linéaire ou ramifié, ayant 1 à 3 atomes de carbone.

La réaction est généralement effectuée dans un solvant organique formant un azéotrope avec l'eau, afin de permettre l'élimination de l'eau formée dans la réaction.

On peut utiliser notamment les mêmes solvants que pour la réaction d'estérification décrite précédemment.

Comme pour la réaction d'estérification précédente, la température est généralement de 50°C à 150°C et en pratique sera souvent la température de reflux du solvant utilisé.

Parmi les acides carboxyliques de formule (II), on peut citer comme composés nouveaux, utilisables comme intermédiaires dans la synthèse des composés soufrés de formule (I) :
- l'acide (2-thiényl)-4 dithia-3,5 pimélique,
- le bis[bis(hydroxycarbonylméthyl thio)méthylène[-2,5 thiophène.

Les composés soufrés de formule (I) conviennent notamment pour la préparation de polythiouréthannes, par réaction entre lesdits composés (I) et au moins un polyisocyanate.

On peut utiliser les composés soufrés de formule (I) en mélanges avec des polythiols connus.

A titre d'exemples de tels polythiols, on peut citer le tétrakis(thioglycolate) de pentaérythrityle, le tétrakis(mercapto-2 propionate) de pentaérythrityle, le tris(thioglycolate) du triméthylolpropane, le tris(mercapto-2 propionate) du triméthylolpropane, le benzène-dithiol-1,2, le benzène-dithiol-1,4, l'hexakis(thioglycolate) du dipentaérythritol, le tris(mercapto-3 propyl)-1,3,5 isocyanurate.

On peut également se référer aux polythiols tels que des composés légers e.g. dimercapto-butane, pentaerithrithiol, et à ceux cités dans le brevet EP-A-0 351 073.

On préfère comme constituant thiol autre que ceux selon l'invention, des monomères saturé acyclique non ester porteur d'au moins trois fonctions réactives à l'égard des isocyanates pour former des liaisons carbonates, parmi lesquels fonctions réactives au moins 40 % en nombre sont des groupes mercaptans SH, la proportion desdites fonctions étant d'au moins 45 % en massepar rapport à la masse molèculaire dudit monomère.

Il n'y a pas de limitation particulière pour les polyisocyanates servant à préparer lesdits polythiouréthannes.

Ce sont des polyisocyanates proprement dits comme par exemple le toluène-diisocyanate, le diisocyanato-4,4' diphénylméthane, le diisocyanato-4,4' diphénylméthane polymérique, l'hexaméthylène diisocyanate, le méthyl-2 diisocyanato-1,5 pentane, l'éthyl-2 diisocyanato-1 ,4 butane, l'isophorone-diisocyanate, le xylylène-diisocyanate, le diisocyanato-4,4' dicyclohexylméthane, le xylylène-diisocyanate hydrogéné.

Ce sont également les trimères à cycle isocyanurate ou les biurets de tels polyisocyanates.

Parmi les polyisocyanates, on préférera ceux dans la formule desquels les fonctions -NCO ne sont pas directement liées à un cycle aromatique, car les polythiouréthannes préparés à partir des polyisocyanates aromatiques ont tendance à être colorés.

D'autre part, il est bien évident que parmi les polyisocyanates, on choisira ceux qui présentent déjà eux-mêmes un indice de réfraction suffisamment élevé, comme par exemple le xylylène-diisocyanate.

Ainsi il est préféré d'utiliser un polyisocyanate ayant un indice de réfraction égal ou supérieur à 1,53.

La réaction entre les composés soufrés de formule (I) et les polyisocyanates se fait dans les conditions habituelles de ce type de réaction.

Généralement on utilise un composé organique de l'étain comme catalyseur, le plus souvent le dilaurate de dibutyl étain.

Les composés soufrés de formule (I) sont utilisés également pour la préparation de polythioéthers par réaction avec au moins un composé présentant au moins deux insaturations éthyléniques.

Comme composés insaturés, on peut citer par exemple le triallylisocyanurate, le triallylcyanurate, le phtalate de diallyle, le divinylbenzène, le styrène, le triméthallylthiocyanurate, le tris-acrylate d'hydroxy-2 éthylcyanurate, le tris-méthacrylate d'hydroxy-2 éthylcyanurate, le tris(allylcarbonate) d'hydroxy-2 éthylcyanurate, le tris(méthallylcarbonate) d'hydroxy-2 éthylcyanurate, le diacrylate du bis(hydroxy-4 phényl)-2,2 propane.

Parmi ces composés insaturés, on préfèrera ceux qui présentent le plus grand nombre d'insaturations éthyléniques et ceux qui possèdent eux-mêmes un indice de réfraction suffisamment élevé.

Si le nombre d'insaturations éthyléniques n'est pas supérieur à 2, on préfèrera mettre en oeuvre un mélange ternaire composé soufré/ composé di-insaturé/triacrylate ou triméthacrylate, afin d'avoir un verre dur. Les triacrylates ou triméthacrylates sont par exemple le triacrylate de triméthylolpropane, le triacrylate du pentaérythritol, le triméthacrylate du pentaérythritol, l'hexa-acrylate du dipentaérythritol, l'hexaméthacrylate du dipentaérythritol.

La réaction entre les composés soufrés de formule (I) et les composés à insaturations éthyléniques se fait avec un excès d'insaturations éthyléniques par rapport aux fonctions thiol.

Il a en effet été mis en évidence une certaine lenteur de la polymérisation, lorsque l'on augmente la quantité de fonctions thiol.

Cette préparation se fait généralement en présence de catalyseurs, tels que les peroxydes et plus généralement les générateurs de radicaux.

Généralement on utilise de 0,1 % à 10 % en poids de catalyseur par rapport au poids du mélange réactionnel.

Les polythiouréthannes et les polythioéthers obtenus à partir des composés soufrés de formule (I) ont des propriétés qui permettent leur utilisation en optique.

En effet ces polymères ont un indice de réfraction supérieur à 1,50 et un nombre d'ABBE supérieur ou égal à 30, ce qui est représentatif d'une faible dispersion.

Ces polymères constituent seul ou en mélange des matériaux trés utile pour de nombreuses utilisations optiques y compris la fabrication de lentille ophtalmique.

Ces matériaux ne se dégradent pas dans le temps, ni à la lumière, ni aux imtempéries (jaunissement ou pertes de propriété mécaniques) et ils présentent une bonne résistance aux chocs à l'abrasion ils sont colorables et facile à démouler.

Parmi les applications optiques, on peut citer la fabrication de disques optiques et de guides d'ondes.

Les exemples qui suivent illustrent l'invention.

### Mode opératoire de la fabrication des lentilles ophtalmiques

Dans un flacon, on mélange 9,4 g (0,05 mole) de xylylène diisocyanate (XDI), 6,86 g (0,024 mole) de bisthioglycolate de benzène diméthylène 1,4 préparé ci-dessus et 2,38 g (0,0172 mole) de TTG. On rajoute 0,02 % en masse de dibutyl-laurate d'étain comme catalyseur de polymérisation.

Après avoir été dégazé, ce mélange est placé entre deux moules en verre préalablement traités pour éviter l'adhésion du polymère sur le verre.

La polymérisation est effectuée selon un cycle de température comprenant 6 h pour passer de la température ambiante à 60°C, puis 6 H de maintien à 80°C, suivie de 3 h de maintien à 130°C.

La lentille ophtalmique est obtenue, transparente et incolore, par démoulage du mélange ainsi polymérisé.

### EXEMPLE 1 :

### Préparation du bis[bis(mercapto-2 éthoxycarbonylméthylthio)méthylène]-1,4 benzène

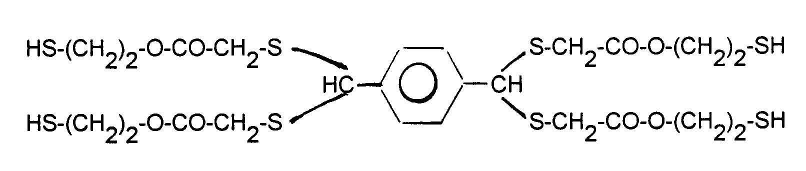

### A) Préparation du bis[bis(hydroxycarbonylméthylthio)méthylène]-1,4benzène

Dans un ballon tricol de 4 titres, muni d'une agitation centrale, d'une gaine thermométrique, d'un "Dean Stark" surmonté d'un réfrigérant sous couverture d'azote, on charge :
- 136,9g (1,0 mol) de téréphtaldéhyde
- 1000 cm³ de toluène.

On chauffe à 70-80°C sous agitation jusqu'à obtention d'un milieu réactionnel homogène.

On coule alors en 2 heures, 372,2 g (4 mol) d'acide thioglycolique
En fin d'addition, le milieu réactionnel est devenu blanc, opaque et très visqueux.

On ajoute alors 1000 cm³ de toluène et on porte à reflux pendant 5 h 30.

On recueille 35 cm³ d'eau dans la partie inférieure du "Dean Stark".

On laisse refroidir le mélange réactionnel jusqu'à température ambiante.
Le précipité formé est essoré, puis lavé à l'aide de 2000 cm³ de cyclohexane, puis est recristallisé dans 6000 cm³ d'eau (les produits non dissous sont éliminés par filtration de la solution à chaud).

Le produit est récupéré par essorage à froid et séché en étuve sous vide à 90°C.

On obtient 392,3 g (rendement de 84 % par rapport au téréphtaldéhyde de départ) d'une poudre blanche dont le point de fusion est de 199-200°C.

Le spectre IR correspond à la structure attendue.

Le dosage des fonctions -COOH par de la soude 0,5N donne 854 milliéquivalents (meq) pour 100 g.

### B) Estérification du bis[bis(hydroxycarbonylméthylthio)méthylène]-1,4 benzène par le thioéthanol

Dans un ballon tricol de 2 litres, muni d'une agitation centrale, d'une gaine thermométrique, d'un "Dean Stark" surmonté d'un réfrigérant sous couverture d'azote, on charge :
- 112 g (0,24 mol) de bis[bis(hydroxycarbonylméthylthio) méthylène]-1,4 benzène
- 187,5g (2,4 mol) de thioéthanol
- 1,1 g d'acide paratoluène sulfonique
- 700 cm³ de toluène.

On chauffe à reflux (109-113°C) sous agitation pendant 7 h 30; le milieu réactionnel est devenu homogène après 1 h 30 de chauffage.

On laisse refroidir le mélange réactionnel jusqu'à température ambiante. On reprend le mélange réactionnel par 400 cm³ d'eau.

La phase toluénique est lavée à l'aide de 2 x 250 cm³ d'eau carbonatée, puis 8 x 300 cm³ d'eau.

On la sèche sur sulfate de sodium. On filtre et on évapore le toluène sous très faible pression.

On obtient 121,5 g (rendement de 71 % par rapport à l'acide de départ) d'un liquide jaune clair, visqueux, ayant un indice de réfraction de 1,595.

On traite ce produit par 350 cm³ de dichlorométhane et 5 g de charbon actif (ébullition pendant 30 min), on filtre et on évapore le solvant sous vide.

On récupère ainsi 118,6 g (rendement de 70 % par rapport à l'acide de départ) d'un liquide incolore, visqueux, ayant un indice de réfraction de 1,595.

Le spectre obtenu par résonance magnétique nucléaire du proton (H¹.RMN) correspond à la structure attendue.

Le dosage des fonctions -SH par la soude 0,1 N donne 521 meq SH/100 g.

### FABRICATION DE LENTILLES OPHTAMIQUES

La composition utilisée comprend :

| | |
|---|---|
| Xylylène diisocyanate (XDI) | 9,4 g (0,05 mole) |
| Trithioglycérine | 3,78 g (0,0266 mole) |
| Bis bis (mercapto-2 éthoxycarbonyl-méthylthio) méthylène 1,4 benzène | 3,29 g (0,005 mole) |
| ((HS-(CH₂)₂-O-CO-CH₂-S)₂HC-pC₆H₄-CH(-S-CH₂-COO-(CH₂)₂-SH)₂) | |

La proportion molaire de trithioglycérine dans le total molaire des monomères est de 84 %.

Le produit obtenu présente les propriétés suivantes :

| | |
|---|---|
| - indice de réfraction | 1,65 |
| - constringence (nombre d'Abbe) | 31 |
| - température de transition vitreuse | 101°C |

### AUTRES FABRICATIONS DE LENTILLES

La composition utilisée comprend :

| | |
|---|---|
| Xylylène diiosocyanate (XDI) | 9,4 g (0,05 mole) |
| Dimercapto-propanol (DMP) | 0,98 g (0,007) mole |
| Bis bis (mercapto-2 éthoxycarbonyl-méthylthio) méthylène-1,4 benzène | |
| ((HS-CH₂-OCO-CH₂-OCO- CH₂-S-)₂ HC-pC₆H₄-CH (-S-CH₂-COO-CH₂SH)₂₎ | |

La proportion molaire de DMP dans le total molaire des monomères est de 26%.

Le produit obtenu présente les propriétés suivantes :

| | |
|---|---|
| - indice de réfraction | 1,62 |
| - constringence (nombre d'Abbe) | 35 |
| - température de transition vitreuse | 100°C |

### EXEMPLE 2:

### Préparation du tétrakis(mercapto-2 éthoxycarbonylméthylthio)1,1,2,2 éthane

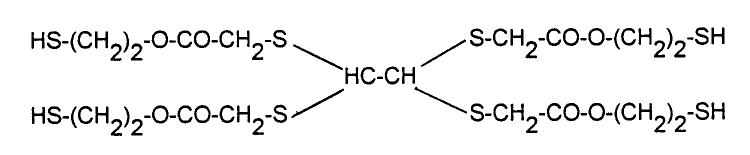

### A) Préparation du tétrakis[hydroxycarbonylméthylthio)1,1,2,2 éthane

Dans un ballon tricol de 250 cm³, muni d'une agitation centrale, d'une gaine thermométrique, d'un réfrigérant sous couverture d'azote et d'une ampoule de coulée, on charge :
- 29 g (0,2 mol) d'une solution aqueuse de glyoxal à 40 % en poids par poids
- 25 cm³ (0,17 mol) d'une solution aqueuse de HCl à 25 % en poids par volume.

Dans le mélange réactionnel maintenu à 5°C , on ajoute goutte à goutte en 55 min, 94,4 (1,02 mol) d'acide thioglycolique distillé.

On laisse revenir le mélange réactionnel jusqu'à température ambiante : il se forme un précipité blanc.

On chauffe le mélange réactionnel hétérogène à 80-90°C pendant 2 h : le mélange reste hétérogène.

Le précipité est essoré, puis lavé à l'aide de 4 x 150 cm³ de d'eau, puis par 200 cm³ d'hexane.

Le produit est séché en étuve sous vide à 80°C.

On obtient 55,7 g (rendement de 71 % par rapport au glyoxal de départ) d'une poudre blanche dont le point de fusion est de 198°C.

Le spectre H¹.RMN correspond à la structure attendue (pureté estimée supérieure à 95 %).

Le dosage des fonctions -COOH par de la soude 0,5N donne 101,4 meq/100 g.

### B) Estérification du tétrakis(hydroxycarbonylméthylthio)1,1,2,2 éthane par le thioéthanol

Dans un ballon tricol de 500 cm³, muni d'une agitation centrale, d'une gaine thermométrique, d'un "Dean Stark" surmonté d'un réfrigérant sous couverture d'azote, on charge :
- 50,76 g (0,13 mol) de tétrakis(hydroxycarbonylméthylthio)1,1,2,2 éthane
- 60,94 g (0,78 mol) de thioéthanol
- 0,59 g d'acide paratoluène sulfonique
- 230 cm³ de toluène.

On chauffe à reflux (106-111°C) sous agitation pendant 15 h ; le milieu réactionnel reste hétérogène.

On laisse refroidir le mélange réactionnel jusqu'à température ambiante.

On essore le précipité; on le sèche. Le solide obtenu (15,7 g) est ocre foncé; l'analyse indique qu'il s'agit de l'acide carboxylique de départ (A).

Le filtrat toluénique est lavé à l'aide de 3 x 100 cm³ d'eau carbonatée à 5 %, puis 4 x 100 cm³ d'eau.

On le sèche sur sulfate de sodium. On filtre et on évapore le toluène sous très faible pression.

On obtient 59,1 g (rendement de 72 % par rapport à l'acide de départ) d'un liquide jaune foncé, ayant un indice de réfraction de 1,591.

On traite ce produit par 100 cm³ de dichlorométhane et 5 g de charbon actif (ébullition pendant 30 min), on filtre et on évapore le solvant sous vide.

On récupère ainsi 53,4 g (rendement de 65 % par rapport à l'acide de départ) d'un liquide incolore, visqueux, ayant un indice de réfraction de 1,593.

Les spectres obtenus par H¹.RMN et IR correspondent à la structure attendue.

Le dosage des fonctions -SH par la soude 0,1 N donne 596 meq SH/100 g.

### FABRICATIOND DE LENTILLES OPHTALMIQUES

La composition utilisée comprend :

| | |
|---|---|
| Xylylène diisocyanate (XDI) | 9,4 g (0,05 mole) |
| Trithioglycérine | 3,7 g (0,0266 mole) |
| Tétrakis (mercapto-2 éthoxycarbonyl-méthylthio) 1,1,2,2, éthane | 2,9g (0,005 mole) |
| ((HS-CH₂-CH₂-O-CO-CH₂-S-)₂ HC-HC (-SCH₂-CO-O-CH₂-SH)₂₎ | |

La proportion molaire de trithioglycérine dans le total molaire des monomères est de 84 %.

Le produit obtenu présente les propriétés suivantes :

| | |
|---|---|
| - indice de réfraction | 1,66 |
| - constringence (nombre d'Abbe) | 32 |
| - température de transition vitreuse | 105°C |

### AUTRES FABRICATIONS DE LENTILLES

La composition utilisée comprend :

| | |
|---|---|
| Xylylène diiosocyanate (XDI) | 9,4 g (0,05 mole) |
| Dimercapto-propanol (DMP) | 0,98 g (0,007) mole |
| Tétrakis (mercapto-2 éthoxycarbonyl-méthylthio)-1,1,2,2,éthane | 11,64g(0,020 mole) |
| ((HS-CH₂-CH₂-O-CO-CH₂-S-)₂ HC-CH (-S-CH₂-CO-O-CH₂-CH₂-SH)₂₎ | |

La proportion molaire de DMP dans le total molaire des monomères est de 26 %.

Le produit obtenu présente les propriétés suivantes :

| | |
|---|---|
| - indice de réfraction | 1,63 |
| - constringence (nombre d'Abbe) | 34 |
| - température de transition vitreuse | 105°C |

### EXEMPLE 3:

### Préparation du (2-thiényl)-4 dithia-3,5 pimélate de bis(mercapto-2 éthyle)

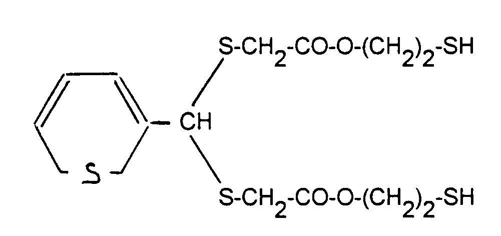

### A) Préparation de l'acide (2-thiényl)-4 dithia-3,5 pimélique

Dans un ballon tricol de 500 cm³, muni d'une agitation centrale, d'une gaine thermométrique, d'une ampoule de coulée tout d'abord, puis d'un "Dean Stark" surmonté d'un réfrigérant sous couverture d'azote, on charge :
- 45,8 g (0,4 mol) de thiophène carbaldéhyde-2
- 250 cm³ de toluène.

Dans le mélange réactionnel maintenu à 15°C, on coule goutte à goutte, en 35 min, 81,1 g (0,88 mol) d'acide thioglycolique.

On laisse revenir le mélange à température ambiante, puis on chauffe à reflux (110-113°C) pendant 2 h 45.

On laisse refroidir le mélange réactionnel jusqu'à température ambiante, puis on évapore le solvant sous pression très réduite.

Le résidu (solide ocre et pâteux) est recristallisé dans 500 cm³ d'eau, est traité par du charbon actif (30 min à 80°C), puis est séché sous pression très réduite en dessicateur (contenant P₂O₅) jusqu'à poids constant.

On obtient 98,2 g (rendement de 88 % par rapport à l'aldéhyde de départ) d'une poudre ocre clair dont le point de fusion est de 115°C.

Les spectres IR et H¹.RMN correspondent à la structure attendue (pureté estimée supérieure à 95 %).

Le dosage des fonctions -COOH par de la soude 0,5N donne 723 meq pour 100 g.

### B) Estérification de l'acide (2-thiényl)-4 dithia-3,5 pimélique par le thioéthanol

Dans un ballon tricol de 500 cm³, muni d'une agitation centrale, d'une gaine thermométrique, d'un "Dean Stark" surmonté d'un réfrigérant sous couverture d'azote, on charge :
- 56 g (0,2 mol) d'acide (2-thiényl)-4 dithia-3,5 pimélique
- 47 g (0,6 mol) de thioéthanol
- 0,46 g d'acide paratoluène sulfonique
- 200 cm³ de toluène.

On chauffe à reflux (99-114°C) sous agitation pendant 3 h 20 ; le milieu réactionnel est devenu homogène pendant la montée de température.

On laisse refroidir le mélange réactionnel jusqu'à température ambiante.

Le mélange réactionnel est lavé à l'aide de 2 x 100 cm³ d'eau carbonatée (à 5 %), puis de 6 x 100 cm³ d'eau.

On le sèche sur sulfate de sodium. On filtre et on évapore le toluène sous très faible pression.

On obtient 78,6 g (rendement de 98,6 % par rapport à l'acide de départ) d'un liquide jaune ayant un indice de réfraction de 1,596.

Une analyse par chromatographie en couche mince indique la présence de plusieurs produits, parmi lesquels on décèle par H¹.RMN majoritairement le produit attendu.

La séparation est effectuée ensuite par chromatographie liquide à haute performance préparative.

### FABRICATIOND DE LENTILLES OPHTALMIQUES

La composition utilisée comprend :

| | |
|---|---|
| Xylylène diisocyanate (XDI) | 9,4 g (0,05 mole) |
| Trithioglycérine | 3,7 g (0,0266 mole) |
| (2-thiényl)-4 dithia-3,5 pimélate de bis (mercapto-2 éthyle) (thiényl - CH (-S-CH₂-CO-O-CH₂-CH₂-SH)₂) | |

La proportion molaire de trithioglycérine dans le total molaire des monomères est de 84 %.

Le produit obtenu présente les propriétés suivantes :

| | |
|---|---|
| - indice de réfraction | 1,65 |
| - constringence (nombre d'Abbe) | 30 |
| - température de transition vitreuse | 95°C |

### EXEMPLE 4:

### Préparation du phényl-4 dithia-3,5 pimélate de bis(mercapto-2 éthyle)

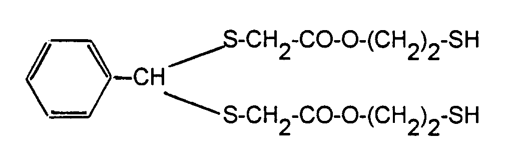

### A) Préparation de l'acide phényl-4 dithia-3,5 pimélique

Dans un ballon tricol de 500 cm³, muni d'une agitation centrale, d'une gaine thermométrique, d'une ampoule de coulée tout d'abord, puis d'un "Dean Stark" surmonté d'un réfrigérant sous couverture d'azote, on charge :
- 47,8 g (0,45 mol) de benzaldéhyde
- 150 cm³ de toluène.

Dans le mélange réactionnel, on coule 82,9 g (0,9 mol) d'acide thioglycolique distillé.

On observe une légère exothermicité : en fin d'addition la température du mélange réactionnel est de 37°C et un solide blanc commence à se former.

On chauffe alors à 50-60°C; le milieu devient limpide.

Après 3 h 20 de maintien à cette température, le mélange réactionnel est devenu blanc, opaque et très visqueux.

On ajoute alors 150 cm³ de toluène et on porte au reflux (110-112°C) pendant 3h : on récupère 7 g d'eau dans la partie inférieure du "Dean Stark".

On laisse refroidir le mélange réactionnel jusqu'à température ambiante.

Le précipité formé est essoré puis lavée par 2 x 200 cm³ d'hexane.

Le produit est essoré et séché sous pression très réduite dans une étuve à 100°C.

On obtient 115,8 g (rendement de 94 % par rapport à l'aldéhyde de départ) d'une poudre blanche dont le point de fusion est de 127°C.

Les spectres IR et H¹.RMN correspondent à la structure attendue.

Le dosage des fonctions -COOH par de la soude 0,5N donne 739 meq pour 100 g.

### B) Estérification de l'acide phényl-4 dithia-3,5 pimélique par le thioéthanol

Dans un ballon tricol de 250 cm³, muni d'une agitation centrale, d'une gaine thermométrique, d'un "Dean Stark" surmonté d'un réfrigérant sous couverture d'azote, on charge :
- 27,23 g (0,1 mol) d'acide phényl-4 dithia-3,5 pimélique
- 15,63 g (0,2 mol) de thioéthanol
- 0,09 g d'acide paratoluène sulfonique
- 140 cm³ de toluène.

On chauffe à reflux (110-112°C) sous agitation pendant 6 h ; le milieu réactionnel est devenu homogène après 30 min.

On laisse refroidir le mélange réactionnel jusqu'à température ambiante.

On reprend le mélange réactionnel par 200 cm³ d'eau.

La phase toluénique est lavée à l'aide de 2 x 150 cm³ d'eau carbonatée (à 5 %), puis de 8 x 150 cm³ d'eau.

On le sèche sur sulfate de sodium. On filtre et on évapore le toluène sous très faible pression.

On obtient 34,1 g (rendement de 86 % par rapport à l'acide de départ) d'un liquide jaune pâle, visqueux ayant un indice de réfraction de 1,596.

Le spectre obtenu par résonance magnétique nucléaire du proton (H¹.RMN) correspond à la structure attendue.

Le dosage des fonctions -SH par la soude 0,1 N donne 330 meq SH/100 g.

### EXEMPLE 5:

### Obtention d'un verre à partir du composé de formule générale (I) (préparé dans l'exemple 1B)

On mélange à température ambiante :
- 60 g de triallylisocyanurate
- 40 g de composé soufré de formule (I) préparé dans l'exemple 1B
- 3 g de triméthyl-3,3,5 perhexanoate de tertiobutyle
Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon un cycle thermique d'environ 30 h 30 : on élève la température à 35°C en 30 min, puis à 40°C en 30 min puis à 50°C en 30 min, puis à 60°C en 30 min, puis à 80°C en 30 min, puis à 92°C en 1 h.

On maintient alors pendant 26 h à 92°C et enfin on élève la température à 100°C pendant 1 h.

On laisse ensuite la température revenir à 20°C.

Le verre obtenu est transparent.

Ses caractéristiques sont les suivantes :

| | |
|---|---|
| -densité | = 1,32 |
| - indice de réfraction | = 1,589 |
| - nombre d'ABBE | = 33 |

### EXEMPLE 6:

### Obtention d'un verre à partir du composé de formule (I) (préparé dans l'exemple 2B)

On mélange à température ambiante :
- 50 g de triallylisocyanurate,
- 50 g de composé soufré de formule (I) préparé dans l'exemple 2B,
- 3 g de triméthyl-3,3,5 perhexanoate de tertiobutyle.

Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon un cycle thermique d'environ 19h : on élève la température à 40°C en 8 min, puis à 60°C en 10 min, puis à 85°C en 1 h; puis on élève la température de 85 à 95°C pendant 16 h et enfin à 105°C en 1 h ; on maintient encore pendant 1 h à 105°C. On laisse ensuite la température revenir à 20°C.

Le verre obtenu est transparent.

Ses caractéristiques sont les suivantes :

| | |
|---|---|
| - densité | = 1,36 |
| - indice de réfraction | = 1,590 |
| - nombre d'ABBE | = 40 |

### EXEMPLE 7:

### Obtention d'un verre à partir du composé de formule (I) (préparé dans l'exemple 3B)

On mélange à température ambiante :
- 1,88 g de xylylène diisocyanate
- 3,54 g de composé soufré de formule (I) préparé dans l'exemple lB,
- 0,0011 g de dilaurate de dibutylétain.

Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon le cycle thermique suivant :
- 3 heures de chauffage pour passer de la température ambiante à 80°C,
- 3 heures de maintien à 80°C,
- 2 heures de chauffage pour passer de 80°C à 130°C,
- 3 heures de maintien à 130°C.

On laisse ensuite la température redescendre à 20°C.

Le verre obtenu est rose et transparent.

Ses caractéristiques sont les suivantes :

| | |
|---|---|
| - indice de réfraction | = 1,624 |
| - nombre d'ABBE | = 31 |

## Revendications

1. Composés soufrés de formule générale (I) : 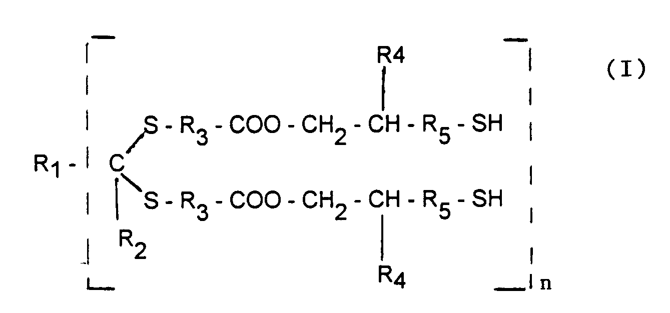 dans laquelle :
- n représente 1 ou 2
- R₁ représente:
. lorsque n = 1 :
+ un radical alkyle, linéaire ou ramifié, ayant 1 à 6 atomes de carbone,
+ un radical phényle,
+ un radical thiényle-2,
. lorsque n = 2 :
+ un valentiel simple,
+ un radical alkylène, linéaire ou ramifié ayant 1 à 4 atomes de carbone,
+ un radical paraphénylène, métaphénylène ou orthophénylène,
+ un radical thiophènediyle-2,4, thiophènediyle-2,5 ou thiophènediyle-2,3,
- R₂ représente :
. un atome d'hydrogène,
. un radical alkyle, linéaire ou ramifié ayant 1 à 6 atomes de carbone,
. un radical phényle,
- R₃ représente un radical alkylène, linéaire ou ramifié, ayant 1 à 3 atomes de carbone,
- R₄ représente :
. un atome d'hydrogène,
. un radical SH,
- R₅ représente :
. un lien valentiel simple,
. un radical méthylène.

2. Composés de formule (I) selon la revendication 1, caractérisés en ce que :
- n représente 1 ou 2,
- R₁ représente :
. lorsque n = 1 :
+ un radical alkyle, linéaire ou ramifié, ayant 1 à 4 atomes de carbone,
+ un radical phényle,
+ un radical thiényle-2,
. lorsque n = 2 :
+ un lien valentiel simple,
+ un radical alkylène, linéaire ou ramifié, ayant 1 à 4 atomes de carbone,
+ un radical paraphénylène,
+ un radical thiophènediyle-2,5,
- R₂ représente :
. un atome d'hydrogène,
. un radical alkyle, linéaire ou ramifié, ayant 1 à 4 atomes de carbone,
. un radical phényle,
- R₃ représente un radical méthylène,
- R₄ représente un atome d'hydrogène,
- R₅ représente un lien valentiel simple.

3. Composés soufrés de formule (I) selon l'une des revendications 1 ou 2, caractérisés en ce qu'ils sont choisis parmi :
- le tétrakis(mercapto-2 éthoxycarbonylméthylthio)-1,1,2,2 éthane,
- le bis[bis(mercapto-2 éthoxycarbonylméthylthio)méthylène]-1,4 benzène,
- le (2-thiényl)-4 dithia-3,5 pimélate de bis(mercapto-2 éthyle),
- le phényl-4 dithia-3,5 pimélate de bis(mercapto-2 éthyle).

4. Procédé de préparation de composés soufrés de formule (I) selon l'une des revendications 1 à 3, caractérisé en ce que l'on effectue une estérification de l'acide carboxylique correspondant de formule (II) : 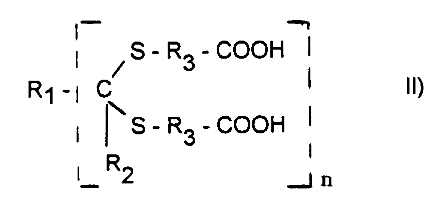 dans laquelle les symboles R₁, R₂, R₃ et n ont les significations indiquées précédemment pour la formule (I), avec un thioalcanol de formule (III) : 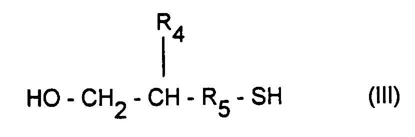 dans laquelle les symboles R₄ et R₅ ont les significations indiquées précédemment pour la formule (I).

5. Procédé selon la revendication 4, caractérisé en ce que l'on opère en présence de catalyseurs tels que les acides protoniques, dans un solvant organique formant un azéotrope avec l'eau et à une température de 50°C à 150°C.

6. Procédé selon l'une des revendications 4 ou 5, caractérisé en ce que les acides carboxyliques de formule (II) sont préparés par réaction d'un aldéhyde ou d'une cétone de formule générale (IV) : 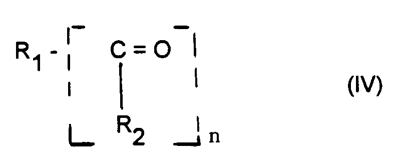 dans laquelle les symboles R₁ et R₂ ont les significations indiquées précédemment pour les composés soufrés de formule (I) et n représente 1 ou 2, avec un acide mercapto-carboxylique de formule générale (V) :
HS - R₃ - COOH (V)
dans laquelle R₃ représente un radical alkylène, linéaire ou ramifié, ayant 1 à 3 atomes de carbone,
dans un solvant organique formant un azéotrope avec l'eau, à une température de 50°C à 150°C.

7. - l'acide (2-thiényl)-4 dithia-3,5 pimélique, et
- le bis[bis(hydroxycarbonylméthyl thio)méthylène]-2,5 thiophène, utilisables comme intermédiaires dans la synthèse des composés soufrés de formule (I) selon l'une des revendications 1 à 3.

8. Polythiouréthannes caractérisés en ce qu'ils sont préparés par réaction entre les composés soufrés de formule (I) selon l'une des revendications 1 à 3 et au moins un polyisocyanate, avantageusement au moins un polyisocyanate tel que le toluénediisocyanate, le diisocyanato-4,4' diphénylméthane, le diisocyanato-4,4' diphénylméthane polymérique, l'examéthylène diisocyanate, le méthyl-2 diisocyanato-1,5 pentane, l'éthyl-2 iisocyanato-1,4 butane, l'isophorone-diisocyanate, le sylilènediisocyanate, le diisocyanato-4,4' dicyclohexylméthane, le xylilène-diisocyanate, le diisocyanato-4,4' dicyclohexylméthane, le xylilène-diisocyanate hydrogéné, les trimères à ce cycle isocyanurate et les biurets de tels polyisocyanates.

9. Polythiouréthannes selon la revendication 8, caractérisés en ce que le polyisocyanate est choisi parmi ceux dans la formule desquels les fonctions -NCO ne sont pas directement lièes à un cycle aromatique.

10. Polythiouréthannes selon l'une des revendications 8 ou 9, caractérisés en ce qu'il comporte, outre au moins un composés soufrés de formule (I) selon l'une des revendications 1 à 3, au moins un monomère avantageusement porteur d'au moins trois fonctions réactives avec les isocyanates pour donner des fonctions carbamates, lesdites fonctions réactives étant de préférence telle que quelle soient constituées d'au moins 40 % de fonction mercaptan (-SH) et avantageusement la masse des dites fonctions étant d'au moins 45 % par rapport à la masse des monomères.

11. Polythiouréthannes selon l'une des revendications 8 à 10, caractérisés en ce que le polyisocyanate est choisi parmi ceux qui ont un indice de réfraction égal ou supérieur à 1,53.

12. Polythioéthers caractérisés en ce qu'ils sont préparés par réaction entre les composés soufrés de formule (I) selon l'une des revendications 1 à 3 et au moins un composé présentant au moins deux insaturations éthyléniques.

13. Polythioéthers selon la revendication 12, caractérisés en ce que le composé à insaturation éthylènique est choisi parmi le triallylisocyanurate, le triallylcyanurate, le phtalate de diallyle, le divinylbenzène, le styrène, le triméthallylthiocyanurate, le tris-acrylate d'hydroxy-2 éthylcyanurate, le tris-méthacrylate d'hydroxy-2 éthylcyanurate, le tris(allylcarbonate) d'hydroxy-2 éthylcyanurate, le tris(méthallylcarbonate) d'hydroxy-2 éthylcyanurate, le diacrylate du bis(hydroxy-4 phényl)-2,2 propane.

14. Polythioéthers selon l'une des revendications 12 ou 13, caractérisés en ce que l'on met en oeuvre un mélange ternaire composé soufré/composé di-insaturé/triacrylate ou triméthacrylate tel que le triacrylate de triméthylolpropane, le triacrylate du pentaérythritol, le triméthacrylate du pentaérythritol, l'hexa-acrylate du dipentaérythritol, l'hexaméthacrylate du dipentaérythritol.

15. Polythioéthers selon l'une des revendications 12 à 14, caractérisés en ce que la réaction entre les composés soufrés de formule (I) et les composés à insaturations éthylèniques se fait avec un excès d'insaturations éthylèniques par rapport aux fonctions thiol.

16. Polythioéthers selon l'une des revendications 12 à 15, caractérisés en ce que leur préparation se fait en présence de catalyseurs, tels que les peroxydes et plus généralement les générateurs de radicaux.

17. Polythioéthers selon l'une des revendications 12 à 16, caractérisés en ce que lors de leur préparation on utilise de 0,1 % à 10 % en poids de catalyseur par rapport au poids du mélange réactionnel.

18. Matériau utile pour application optique comportant au moins pour moitié, de préférence pour 3/4, avantageusement pour 9/10 des polymères formés à partir des composés de la revendication 1, les polymères étant avantageusement choisi parmi ceux visé aux revendication 8 à 17.

## Claims

1. Sulphur compounds of general formula (I): 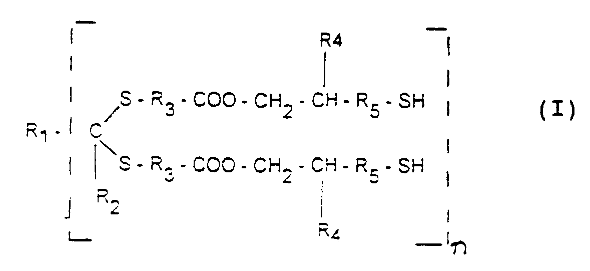 in which:
- n denotes 1 or 2
- R₁ denotes:
when n = 1:
+ a linear or branched alkyl radical containing 1 to 6 carbon atoms,
+ a phenyl radical,
+ a 2-thienyl radical,
when n = 2:
+ a single valency,
+ a linear or branched alkylene radical containing 1 to 4 carbon atoms,
+ a para-phenylene, meta-phenylene or ortho-phenylene radical,
+ a 2,4-thiophenediyl, 2,5-thiophenediyl or 2,3-thiophenediyl radical,
- R₂ denotes:
a hydrogen atom,
a linear or branched alkyl radical containing
1 to 6 carbon atoms,
a phenyl radical,
- R₃ denotes a linear or branched alkylene radical containing 1 to 3 carbon atoms,
- R₄ denotes:
a hydrogen atom,
an SH radical,
- R₅ denotes:
a single valency bond,
a methylene radical.

2. Compounds of formula (I) according to Claim 1, characterized in that :
- n denotes 1 or 2
- R₁ denotes:
when n = 1:
+ a linear or branched alkyl radical containing 1 to 4 carbon atoms,
+ a phenyl radical,
+ a 2-thienyl radical,
when n = 2:
+ a single valency bond,
+ a linear or branched alkylene radical containing 1 to 4 carbon atoms,
+ a para-phenylene radical,
+ a 2,5-thiophenediyl radical,
- R₂ denotes:
a hydrogen atom,
a linear or branched alkyl radical containing
1 to 4 carbon atoms,
a phenyl radical,
- R₃ denotes a methylene radical,
- R₄ denotes a hydrogen atom,
- R₅ denotes a single valency bond.

3. Sulphur compounds of formula (I) according to either of Claims 1 and 2, characterized in that they are chosen from :
- 1,1,2,2-tetrakis(2-mercaptoethoxycarbonylmethylthio)ethane,
- 1,4-bis[bis(2-mercaptoethoxycarbonylmethylthio)methylene]benzene,
- di(2-mercaptoethyl) 4-(2-thienyl)-3,5-dithiapimelate,
- di(2-mercaptoethyl) 4-phenyl-3,5-dithiapimelate.

4. Process for the preparation of sulphur compounds of formula (I) according, to one of Claims 1 to 3, characterized in that an esterification is performed of the carboxylic acid corresponding to formula (II) : 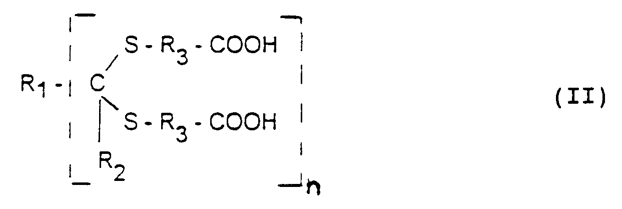 in which the symbols R₁, R₂, R₃ and n have the meanings shown above for formula (I), with a thioalkanol of formula (III): 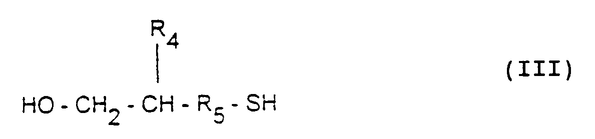 in which the symbols R₄ and R₅ have the meanings given above for formula (I).

5. Process according to Claim 4, characterized in that the operation is carried out in the presence of catalysts such as protonic acids, in an organic solvent forming an azeotrope with water and at a temperature of 50°C to 150°C.

6. Process according to either of Claims 4 and 5, characterized in that the carboxylic acids of formula (II) are prepared by reaction of an aldehyde or of a ketone of general formula (IV): 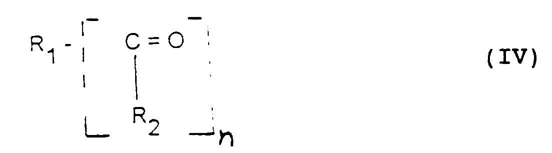 in which the symbols R₁ and R₂ have the meanings indicated above for the sulphur compounds of formula (I) and n denotes 1 or 2, with a mercaptocarboxylic acid of general formula (V):
HS-R₃-COOH (V)
in which R₃ denotes a linear or branched alkylene radical containing 1 to 3 carbon atoms,
in an organic solvent forming an azeotrope with water, at a temperature of 50°C to 150°C.

7. - 4-(2-Thienyl)-3,5-dithiapimelic acid, and
- 2,5-bis[bis(hydroxycarbonylmethylthio)methylene]thiophene, which are capable of being employed as intermediates in the synthesis of the sulphur compounds of formula (I) according to one of Claims 1 to 3.

8. Polythiourethanes characterized in that they are prepared by reaction between the sulphur compounds of formula (I) according to one of Claims 1 to 3 and at least one polyisocyanate, advantageously at least one polyisocyanate such as toluene diisocyanate, 4,4'-diisocyanatodiphenylmethane, polymeric 4,4' -diisocyanatodiphenylmethane, hexamethylene diisocyanate, 2-methyl-1,5-diisocyanatopentane, 2-ethyl-1,4-diisocyanatobutane, isophorone diisocyanate, sylilene diisocyanate, 4,4'-diisocyanatodicyclohexylmethane, xylylene diisocyanate, 4,4'-diisocyanatodicyclohexylmethane, hydrogenated xylylene diisocyanate, trimers containing this isocyanurate ring and biurets of such polyisocyanates.

9. Polythiourethanes according to Claim 8, characterized in that the polyisocyanate is chosen from those in whose formula the -NCO functional groups are not bonded directly to an aromatic ring.

10. Polythiourethanes according to either of Claims 8 and 9, characterized in that, besides at least one sulphur compound of formula (I), according to one of Claims 1 to 3, they comprise at least one monomer advantageously bearing at least three functional groups that react with isocyanates to give carbamate functional groups, the said reactive functional groups being preferably such that they consist of at least 40 % of mercaptan (-SH) functional group and advantageously the mass of the said functional groups being at least 45 % relative to the mass of the monomers.

11. Polythiourethanes according to one of Claims 8 to 10, characterized in that the polyisocyanate is chosen from those which have a refractive index equal to or higher than 1.53.

12. Polythioethers characterized in that they are prepared by reaction between the sulphur compounds of formula (I) according to one of Claims 1 to 3 and at least one compound containing at least two ethylenic unsaturations.

13. Polythioethers according to Claim 12, characterized in that the compound containing ethylenic unsaturation is chosen from triallyl isocyanurate, triallyl cyanurate, diallyl phthalate, divinylbenzene, styrene, trimethallyl thiocyanurate, 2-hydroxyethylcyanurate triacrylate, 2-hydroxyethylcyanurate trimethacrylate, 2-hydroxyethylcyanurate tris(allylcarbonate), 2-hydroxyethylcyanurate tris (methallylcarbonate) and 2,2-bis(4-hydroxyphenyl)propane diacrylate.

14. Polythioethers according to either of Claims 12 and 13, characterized in that a ternary mixture of sulphur compound/diunsaturated compound/triacrylate or trimethacrylate such as trimethylolpropane triacrylate, pentaerythritol triacrylate, pentaerythritol trimethacrylate, dipentaerythritol hexaacrylate or dipentaerythritol hexamethacrylate is used

15. Polythioethers according to one of Claims 12 to 14, characterized in that the reaction between the sulphur compounds of formula (I) and the compounds containing ethylenic unsaturations takes place with an excess of ethylenic unsaturations relative to the thiol functional groups.

16. Polythioethers according to one of Claims 12 to 15, characterized in that their preparation takes place in the presence of catalysts such as peroxides and, more generally, radical-generators.

17. Polythioethers according to one of Claims 12 to 16, characterized in that during their preparation 0.1 % to 10 % by weight of catalyst is employed relative to the weight of the reaction mixture.

18. Material usable for optical application, comprising, at least as far as half, preferably 3/4, advantageously 9/10 is concerned, polymers formed from the compounds of Claim 1, the polymers being advantageously chosen from those referred to in Claims 8 to 17.

## Patentansprüche

1. Schwefelhaltige Verbindungen der allgemeinen Formel (I): 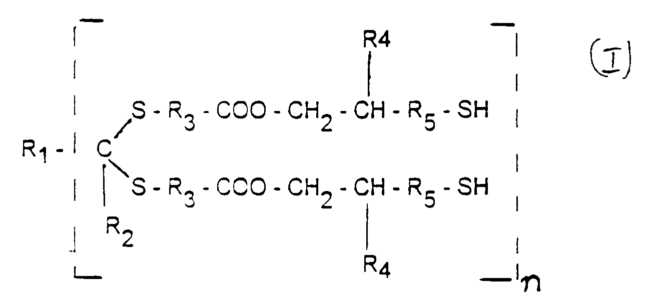 in der
- n 1 oder 2 bedeutet,
- R₁ wenn n = 1:
für eine lineare oder verzweigte Alkylgruppe mit 1 bis 6 Kohlenstoffatomen, eine Phenylgruppe oder eine 2-Thienylgruppe steht,
wenn n = 2:
eine Einfachbindung, eine lineare oder verzweigte Alkylengruppe mit 1 bis 4 Kohlenstoffatomen, eine Paraphenylen-, Metaphenylen- oder Orthophenylengruppe oder eine Thiophen-2,4-diyl-, Thiophen-2,5-diyl- oder Thiophen-2,3-diylgruppe bedeutet,
- R₂ für ein Wasserstoffatom, eine lineare oder verzweigte Alkylgruppe mit 1 bis 6 Kohlenstoffatomen oder eine Phenylgruppe steht,
- R₃ eine lineare oder verzweigte Alkylengruppe mit 1 bis 3 Kohlenstoffatomen bedeutet,
- R₄ ein Wasserstoffatom oder eine Gruppe SH bedeutet und
- R₅ eine Einfachbindung oder eine Methylengruppe bedeutet.

2. Verbindungen der Formel (I) nach Anspruch 1, dadurch gekennzeichnet, daß
- n 1 oder 2 ist,
- R₁ wenn n = 1:
für eine lineare oder verzweigte Alkylgruppe mit 1 bis 4 Kohlenstoffatomen, eine Phenylgruppe oder eine 2-Thienylgruppe steht,
wenn n = 2:
eine Einfachbindung, eine lineare oder verzweigte Alkylengruppe mit 1 bis 4 Kohlenstoffatomen, eine Paraphenylengruppe oder eine Thiophen-2,5-diylgruppe bedeutet,
- R₂ für ein Wasserstoffatom, eine lineare oder verzweigte Alkylgruppe mit 1 bis 4 Kohlenstoffatomen oder eine Phenylgruppe steht,
- R₃ eine Methylengruppe bedeutet,
- R₄ ein Wasserstofftom bedeutet und
- R₅ eine Einfachbindung bedeutet.

3. Schwefelhaltige Verbindungen der Formel (I) nach einem der Ansprüche 1 oder 2, dadurch gekennzeichnet, daß sie ausgewählt sind aus:
- 1,1,2,2,-Tetrakis(2-mercapto-ethoxycarbonylmethylthio)-ethan,
- 1,4-Bis[bis(2-mercapto-ethoxycarbonylmethylthio)-methylen] -benzol,
- Bis(2-mercapto-ethyl)-4-(2-thienyl)-3,5-dithiapimelat,
- Bis(2-mercapto-ethyl)-4-phenyl-3,5-dithiapimelat.

4. Verfahren zur Herstellung von schwefelhaltigen Verbindungen der Formel (I) nach einem der Ansprüche 1 bis 3, dadurch gekennzeichnet, daß man die entsprechende Carbonsäure der Formel (II) 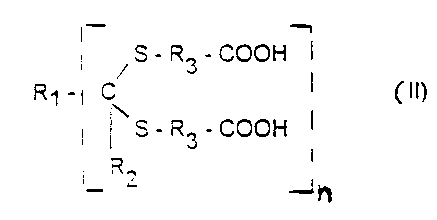 in der R₁, R₂, R₃ und n die zuvor für die Formel (I) angegebenen Bedeutungen haben, mit einem Thioalcanol der Formel (III) 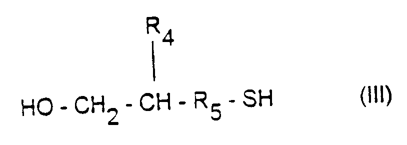 in der R₄ und R₅ die zuvor für die Formel (I) angegebenen Bedeutungen haben, verestert.

5. Verfahren nach Anspruch 4, dadurch gekennzeichnet, daß man in Anwesenheit von Katalysatoren, wie Protonsäuren, in einem organischen Lösungsmittel, das mit Wasser ein Azeotrop bildet, und bei einer Temperatur von 50 bis 150°C arbeitet.

6. Verfahren nach einem der Ansprüche 4 oder 5, dadurch gekennzeichnet, daß die Carbonsäuren der Formel (II) durch Reaktion eines Aldehyds oder eines Ketons der allgemeinen Formel (IV) 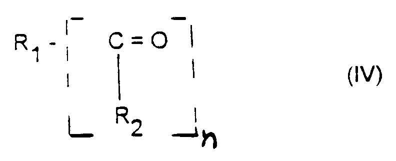 in der R₁ und R₂ die zuvor für die schwefelhaltigen Verbindungen der Formel (I) angegebenen Bedeutungen haben und n 1 oder 2 ist, mit einer Mercapto-carbonsäure der allgemeinen Formel (V)
HS - R₃ - COOH (V),
in der R₃ eine lineare oder verzweigte Alkylengruppe mit 1 bis 3 Kohlenstoffatomen bedeutet, in einem organischen Lösungsmittel, das mit Wasser ein Azeotrop bildet, bei einer Temperatur von 50 bis 150°C hergestellt werden.

7. 4-(2-Thienyl)-3,5-dithia-pimelinsäure und
- 2,5-Bis[bis(hydroxycarbonylmethylthio)methylen]thiopen,
brauchbar als Zwischenprodukte bei der Synthese von schwefelhaltigen Verbindungen der Formel (I) nach einem der Ansprüche 1 bis 3.

8. Polythiourethane, dadurch gekennzeichnet, daß sie durch Reaktion zwischen den schwefelhaltigen Verbindungen der Formel (I) nach einem der Ansprüche 1 bis 3 und mindestens einem Polyisocyanat, vorteilhafterweise mindestens einem Polyisocyanat, wie Toluoldiisocyanat, Diphenylmethan-4,4'-diisocyanat, polymeres Diphenylmethan-4,4'-diisocyanat, 2-Methylpentan-1,5-diisocyanat, 2-Ethylbutan-1,4-diisocyanat, Isophoron-diisocyanat, Sylilen-diisocyanat, Dicyclohexylmethan-4,4'-diisocyanat, Xylilen-diisocyanat, Dicyclohexylmethan-4,4'-diisocyanat, hydriertes Xylilen-diisocyanat, die Trimeren mit diesem Isocyanuratring und die Biuret-Verbindungen derartiger Polyisocyanate, hergestellt werden.

9. Polythiourethane nach Anspruch 8, dadurch gekennzeichnet, daß das Polyisocyanat unter denjenigen ausgewählt wird, bei denen die Gruppen -NCO nicht direkt an einen aromatischen Kern gebunden sind.

10. Polythiourethane nach einem der Ansprüche 8 oder 9, dadurch gekennzeichnet, daß sie zusätzlich zu mindestens einer schwefelhaltigen Verbindung der Formel (I) nach einem der Ansprüche 1 bis 3 mindestens ein Monomer enthalten, das vorteilhafterweise mindestens drei reaktionsfähige Gruppen trägt, die mit den Isocyanaten unter Bildung von Carbamatgruppen reagieren, wobei die reaktionsfähigen Gruppen vorzugsweise mindestens zu 40 % Mercaptangruppen (-SH) sind und vorteilhafterweise die Masse dieser Gruppen mindestens 45 %, bezogen auf die Masse der Monomeren, ausmacht.

11. Polythiourethane nach einem der Ansprüche 8 bis 10, dadurch gekennzeichnet, daß das Polyisocyanat unter denjenigen ausgewählt wird, die einen Brechungsindex von 1,53 oder darüber besitzen.

12. Polythioether, dadurch gekennzeichnet, daß sie durch Reaktion zwischen den schwefelhaltigen Verbindungen der Formel (I) nach einem der Ansprüche 1 bis 3 und mindestens einer Verbindung, die mindestens zwei ethylenisch ungesättigte Bindungen enthält, hergestellt werden.

13. Polythioether nach Anspruch 12, dadurch gekennzeichnet, daß die ethylenisch ungesättigte Verbindung ausgewählt wird aus Triallylisocyanurat, Triallylcyanurat, Diallylphthalat, Divinylbenzol, Styrol, Trimethallythiocyanurat, 2-Hydroxyethylcyanurat-tris(acrylat), 2-Hydroxy-ethylcyanurat-tris(methacrylat), 2-Hydroxy-ethylcyanurat-tris(allylcarbonat), 2-Hydroxyethylcyanurat-tris(methallylcarbonat) und 2,2-Bis(4-hydroxyphenyl) propandiacrylat.

14. Polythioether nach einem der Ansprüche 12 oder 13, dadurch gekennzeichnet, daß man ein ternäres Gemisch aus schwefelhaltiger Verbindung, zweifach ungesättigter Verbindung und Triacrylat oder Trimethacrylat, wie Trimethylolpropan-triacrylat, Pentaerythrit-triacrylat, Pentaerythrit-trimethacrylat, Dipentaerythrit-hexaacrylat oder Dipentaerythrit-hexamethacrylat einsetzt.

15. Polythioether nach einem der Ansprüche 12 bis 14, dadurch gekennzeichnet, daß die Reaktion zwischen den schwefelhaltigen Verbindungen der Formel (I) und den ethylenisch ungesättigten Verbindungen mit einem Überschuß an ethylenisch ungesättigten Bindungen, bezogen auf die Thiolgruppen, erfolgt.

16. Polythioether nach einem der Ansprüche 12 bis 15, dadurch gekennzeichnet, daß ihre Herstellung in Gegenwart von Katalysatoren, wie Peroxide und allgemeiner Radikalbildner, erfolgt.

17. Polythioether nach einem der Ansprüche 12 bis 16, dadurch gekennzeichnet, daß man bei ihrer Herstellung 0,1 bis 10 Gew.-% Katalysator, bezogen auf das Gewicht des Reaktionsgemisches, verwendet.

18. Für optische Anwendung brauchbarer Werkstoff, der mindestens zur Hälfte, vorzugsweise zu 3/4 und vorteilhafter zu 9/10, aus Polymeren, hergestellt ausgehend von den Verbindungen des Anspruchs 1, besteht, wobei die Polymeren vorteilhafterweise unter denen der Ansprüche 8 bis 17 ausgewählt worden sind.
